(19) Europäisches Patentamt
European Patent Office
Office européen des brevets

(11) Veröffentlichungsnummer : **0 146 642**
**B1**

(12) # EUROPÄISCHE PATENTSCHRIFT

(45) Veröffentlichungstag der Patentschrift : **20.01.88**

(51) Int. Cl.⁴ : **C 07 D405/12**, C 07 D239/95, C 07 C127/26

(21) Anmeldenummer : **83111671.0**

(22) Anmeldetag : **22.11.83**

(54) **Verfahren zur Herstellung von 4-Amino-6,7-dimethoxy-2-(4-(furo-2-yl)-piperazin-1-yl)-chinazolin und dessen physiologisch annehmbarer Salze.**

(43) Veröffentlichungstag der Anmeldung : **03.07.85 Patentblatt 85/27**

(45) Bekanntmachung des Hinweises auf die Patenterteilung : **20.01.88 Patentblatt 88/03**

(84) Benannte Vertragsstaaten :
**AT BE CH DE FR GB IT LI LU NL SE**

(56) Entgegenhaltungen :
**EP-A- 0 034 421**
**EP-A- 0 037 971**
**WO-A-79 /001 66**
**NL-A- 7 902 400**

(73) Patentinhaber : **LUDWIG HEUMANN & CO GMBH**
**Heideloffstrasse 18-28 Postfach 2260**
**D-8500 Nürnberg (DE)**

(72) Erfinder : **Schickaneder, Helmut, Dr. Dipl.-Chem.**
**Moosäcker 25**
**D-8501 Eckental (DE)**
Erfinder : **Ahrens, Kurt-Henning, Dr.**
**Praterstrasse 9**
**D-8500 Nürnberg (DE)**

(74) Vertreter : **Kraus, Walter, Dr. et al**
**Patentanwälte Kraus, Weisert & Partner Thomas-Wimmer-Ring 15**
**D-8000 München 22 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 146 642 B1

Jouve, 18, rue St-Denis, 75001 Paris, France

**Beschreibung**

Die Erfindung bezieht sich auf ein neues Verfahren zur Herstellung von 4-Amino-6,7-dimethoxy-2-[4-(furo-2-yl)-piperazin-1-yl]-chinazolin und dessen physiologisch annehmbarer Salze. Diese als Prazosin bezeichnete Verbindung ist bereits aud den US-Patentschriften 3 511 836 und 3 669 968 bekannt. Sie wird als Regulator des kardiovaskulären Systems und insbesondere zur Senkung des Blutdrucks bei Hypertonikern eingesetzt.

Die vorliegende Erfindung bezieht sich außerdem auf die neue Verbindung N"-Cyano-N'-(3,4-dimethoxyphenyl)-0-phenylisoharnstoff.

Die NL-A-79 02 400 beschreibt ein Verfahren zur Herstellung von 4-Amino-6,7-dimethoxy-2-[4-(furo-2-yl)-piperazin-1-yl]-chinazolin (Prazosin), bei dem 3,4-Dimethoxyanilin mit S,S-Dimethyl-N-cyano-dithioimidocarbonat zum N-Cyano-N'-(3,4-dimethoxyphenyl)-S-methylisothioharnstoff umgesetzt, anschließend mit 1-(Furo-2-yl) piperazin zum 4-(Furo-2-yl)-piperazin-1-[N-cyano-N'-(3,4-dimethoxyphenyl)]-carboximidamid kondensiert und dann bei 180 °C zum 4-Amino-6,7-dimethoxy-2-[4-(furo-2-yl)-piperazin-1-yl]-chinazolinring geschlossen wird.

Der Erfindung liegt die Aufgabe zugrunde, die bekannten Verfahren zur Herstellung von Prazosin zu verbessern und insbesondere ein neues Verfahren zu dessen Herstellung zur Verfügung zu stellen, welches unter Verwendung billiger und reaktiverer Stoffe durchgeführt werden kann.

Diese Aufgabe wird erfindungsgemäß dadurch gelöst, daß man

a) 3,4-Dimethoxyanilin der Formel II

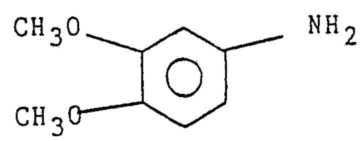

(II)

mit äquimolaren Mengen N-Cyanodiphenylimidocarbonat der Formel III

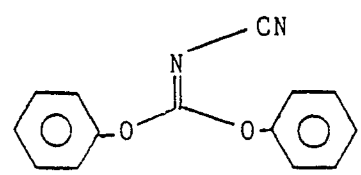

(III)

in einem Alkanol als Lösungsmittel, wobei die Reaktionstemperatur zwischen 20 °C und Rückflußtemperatur des Lösungsmittels beträgt und die Reaktionsdauer, je nach Temperatur, 1 bis 2 Stunden beträgt, zu N"-Cyano-N'-(3,4-dimethoxyphenyl)-O-phenylisoharnstoff der tautomeren Formeln IV

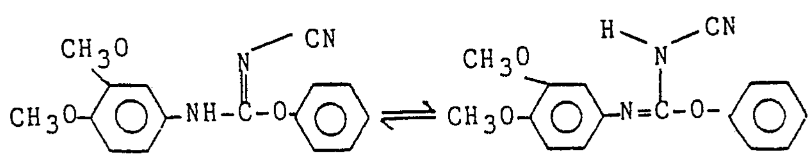

(IV)

umsetzt ;

b) die erhaltene Verbindung der tautomeren Formeln IV mit Piperazin im Überschuß in einem Alkanol, wobei die Reaktionstemperatur zwischen 20 °C und 82 °C und die Reaktionszeit 0,5 bis 1 Stunde beträgt, zu dem Piperazino-1-N"-cyano-N'-(3,4-dimethoxyphenyl) carboximidamid der tautomeren Formeln V

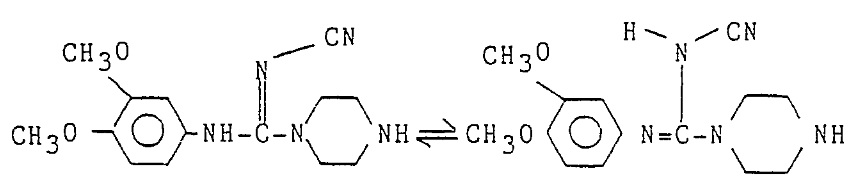

(V)

umsetzt ;

c) anschließend mit einer Säure unter sauren Bedingungen bei einer Temperatur von 20 °C bis 100 °C und einer Reaktionsdauer von 20 bis 120 Minuten zu dem Salz des 4-Amino-6,7-dimethoxy-2-piperazinochinazolins der Formel VI

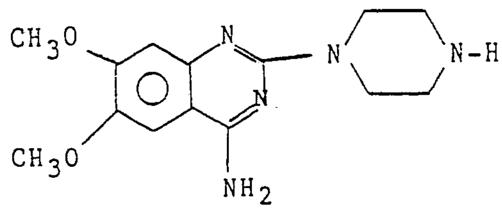

(VI)

cyclisiert ;

d) aus dem in Stufe c) erhaltenen Salz durch Umsetzung mit einem 2- bis 12-fachen molaren Überschuß von wäßrigem Alkali bei Raumtemperatur die freie Base VI gewinnt ;

e) die freie Base mit 2-Furancarbonsäurechlorid in einem Alkanol bei 25 °C zu 4-Amino-6,7-dimethoxy-2 [4-(furo-2-yl)-piperazin-1-yl]-chinazolin-Hydrochlorid umsetzt ; und gegebenenfalls

f) das in Stufe e) erhaltene Hydrochlorid in an sich bekannter Weise in ein anderes physiologisch annehmbares Salz umwandelt.

Der Erfindung liegt die überraschende Entdeckung zugrunde, daß bei der Umsetzung von 3,4-Dimethoxyanilin mit N-Cyanodiphenylimidocarbonat ein neues N''-Cyano-N'-(3,4-dimethoxyphenyl)-O-phenylisoharnstoffderivat entsteht, das sich als Zwischenprodukt dazu eignet, Prazosin unter milden Reaktionsbedingungen — falls gewünscht, in einem Eintopfverfahren — durch einfache Gewinnungs- und Reinigungsverfahren in verbesserter Ausbeute herzustellen. Außerdem stellt die Verwendung des billigen und reaktiveren N-Cyanodiphenylimidocarbonats anstelle des teureren S,S-Dimethyl-N-cyanodithioimidocarbonats einen wirtschaftlichen Vorteil dar.

Das vorliegende Verfahren ist weiterhin vorteilhaft, da im Gegensatz zum oben erwähnten Verfahren kein toxisches und übelriechendes Methylmercaptan freigesetzt wird.

Im folgenden wird das erfindungsgemäße Verfahren anhand der einzelnen Stufen näher beschrieben.

I. Stufe (a) :

In der ersten Stufe setzt man 3,4-Dimethoxyanilin der Formel II

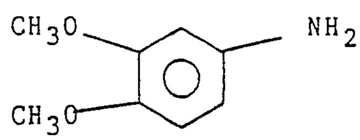

(II)

mit äquimolaren Mengen N-Cyanodiphenylimidocarbonat der Formel III

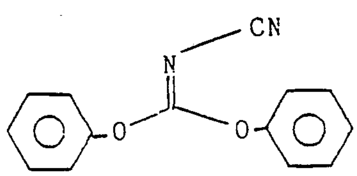

(III)

[R.L. Webb, C.S. Labaw, J. Heterocyclic. Chem. 19, 1205 (1982)] um.

Die Umsetzung erfolgt in einem Alkanol, zum Beispiel Isopropanol, wobei die Reaktionstemperatur zwischen 20 °C und Rückflußtemperatur des Lösungsmittels beträgt. Die Reaktionsdauer beträgt je nach Temperatur 1 bis 2 Stunden.

Zur quantitativen Herstellung von N''-Cyano-N'-(3,4-dimethoxyphenyl)-O-phenylisoharnstoff der tautomeren Formeln IV

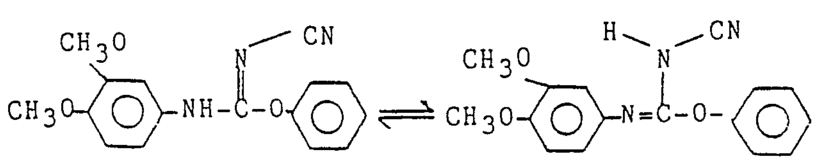

(IV)

läßt man die Reaktion 80 Minuten in Isopropanol bei Rückflußtemperatur ablaufen.

Wie oben angegeben, kann N"-Cyano-N'-(3,4-dimethoxyphenyl)-O-phenylisoharnstoff je nach Lösungsmittelbedingung in den tautomeren Formeln IV vorliegen ; deshalb umfaßt das erfindungsgemäße Verfahren auch die Herstellung der zweiten isomeren Form.

II. Stufe (b) :

In der zweiten Stufe reagiert N"-Cyano-N'-(3,4-dimethoxyphenyl)-O-phenylisoharnstoff der tautomeren Formeln IV mit überschüssigem Piperazin quantitativ zum Piperazino-1-[N"-cyano-N'-(3,4-dimethoxyphenyl)] carboximidamid der tautomeren Formeln V.

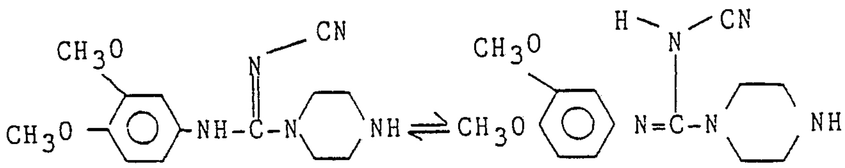

(V)

Die Umsetzung führt man in einem Alkanol durch, wobei Piperazin als Reaktionspartner im Überschuß, vorzugsweise im Molverhältnis von 1 zu 3, zugesetzt wird. Als Lösungsmittel wird Isopropanol bevorzugt, wobei die Reaktionstemperatur zwischen 20 °C und 82 °C, vorzugsweise 82 °C, beträgt. Die Reaktionszeit beträgt je nach Temperaturwahl 1/2 bis 1 Stunde.

III. Stufe (c) :

Die Umwandlung von Piperazino-1-[N"-cyano-N'-(3,4-dimethoxyphenyl)]-carboximidamid der tautomeren Formeln V zum 4-Amino-6,7-dimethoxy-2-piperazinochinazolin der Formel VI

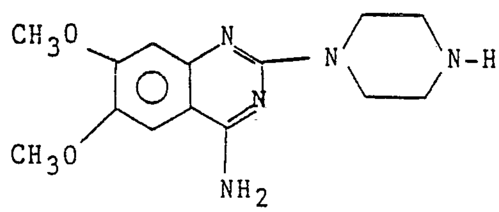

(VI)

erfolgt durch Cyclisierung unter sauren Bedingungen und unter Verwendung beliebiger Säuren mit Einschluß von Lewis-Säuren. Sehr gut geeignet ist zum Beispiel eine 10 %ige wäßrige oder isopropanolische HCl-Lösung, wobei die HCl-Lösung beispielsweise in einem 10 fachen molaren Überschuß eingesetzt wird. Die Cyclisierungsreaktion erfolgt in einem Temperaturbereich von 20 °C bis 100 °C, vorzugsweise in einem Bereich von 50 °C bis 100 °C, insbesondere bei 50 °C. Die Reaktionsdauer liegt je nach Temperaturwahl bei 20 bis 120 Minuten.

Wird die Cyclisierung in Isopropanol durchgeführt, so leitet man in die Reaktionslösung die 2- bis 10 fache molare Menge HCl-Gas und läßt bei 50 °C 1,5 h reagieren.

In dieser Stufe wird das Salz der verwendeten Säure, zum Beispiel das Hydrochlorid, erhalten.

Wie oben erwähnt, lassen sich die Reaktionsstufen a) bis c) — falls gewünscht — in einem Eintopfverfahren durchführen.

IV. Stufe (d) :

Die Freisetzung und Isolierung der freien Base der Formel VI aus dem in Stufe c) erhaltenen Salz, zum Beispiel dem Hydrochlorid, erfolgt durch Zugabe eines 2- bis 12 fachen molaren Überschusses von wäßrigem Alkali, zum Beispiel Natronlauge, bei Raumtemperatur.

V. Stufe (e) :

Die Acylierungsreaktion von 4-Amino-6,7-dimethoxy-2-piperazinochinazolin der Formel VI erfolgt mit 2-Furancarbonsäurechlorid zum 4-Amino-6,7-dimethoxy-2-[4-(furo-2-yl)-piperazin-1-yl] chinazolin-Hydrochlorid der Formel I in einem Alkanol, wie Methanol, Ethanol oder Isopropanol, bei 25 °C in fast quantitativer Ausbeute.

Das in Stufe e) erhaltene Hydrochlorid des 4-Amino-6,7-dimethoxy-2-[4-(furo-2-yl)-piperazin-1-yl] chinazolins kann gewünschtenfalls in an sich bekannter Weise in ein anderes physiologisch annehmbares Salz umgewandelt werden. Dieses Salz kann sich zum Beispiel von einer Mineralsäure, wie Brom- und Jodwasserstoffsäure, Phosphorsäure, Metaphosphorsäure, Salpetersäure oder Schwefelsäure, oder von einer organischen Säure, wie Ameisensäure, Essigsäure, Propionsäure, Phenylessigsäure, Weinsäure, Zitronensäure, Fumarsäure, Methansulfonsäure, Pamoasäure etc., herleiten. Hierzu wird das in Stufe e) erhaltene Hydrochlorid mit Alkali wieder in die Base der Formel I zurückgeführt, und die Base wird sodann in an sich bekannter Weise mit der entsprechenden Säure zu dem gewünschten physiologisch annehmbaren Salz umgesetzt.

Die nach dem erfindungsgemäßen Verfahren erhaltene Verbindung kann zur Verabreichung in gleicher Weise formuliert werden, wie es für Prazosin und dessen Salze, insbesondere das Hydrochlorid, bereits bekannt ist.

Überraschenderweise wurde festgestellt, daß die als Zwischenprodukt bei dem erfindungsgemäßen Verfahren entstehende Verbindung N''-Cyano-N'-(3,4-dimethoxyphenyl)-O-phenylisoharnstoff eine eigene blutdrucksenkende Wirkung besitzt. Diese wurde an spontan-hypertensiven Ratten (Fa. Dr. Ivanovas, Kisslegg) nachgewiesen. Die Blutdruckmessung erfolgte unblutig mit Hilfe eines BP-Recorders (Typ 8005, w + w electronic AG, Basel).

An gesunden Ratten (SIC50, Fa. Dr. Ivanovas, Kisslegg, 160 bis 180 g) wurde ein mittlerer systolischer Blutdruckwert von 166,6 ± 8,0 mbar (125 ± 6 mmHg) (n = 10) ermittelt. Die blutdrucksenkende Wirkung der o. g. Verbindung unter Berücksichtigung des normalen systolischen Wertes in % angegeben, wobei eine 100 %ige Wirkung die Senkung des Blutdrucks auf den normalen Wert von 125 mmHg bedeutet.

Dabei bewirkte N''-Cyano-N'-(3,4-dimethoxyphenyl)-O-phenylisoharnstoff bei einer Dosierung von 3 mg/kg p. o. eine 24 %ige, bei einer Dosierung von 10 mg/kg p. o. eine 42 %ige Normalisierung des systolischen Blutdrucks der spontan-hypertensiven Ratten.

Die Verbindung N''-Cyano-N'-(3,4-dimethoxyphenyl)-O-phenylisoharnstoff der tautomeren Formeln IV

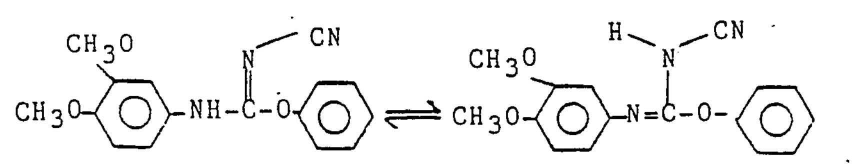

(IV)

ist daher ebenfalls ein Gegenstand der vorliegenden Erfindung.

Die Erfindung wird in dem Beispiel erläutert.

## Beispiel

Herstellung von 4-Amino-6,7-dimethoxy-2-[4-(furo-2-yl)-piperazin-1-yl] chinazolin-Hydrochlorid

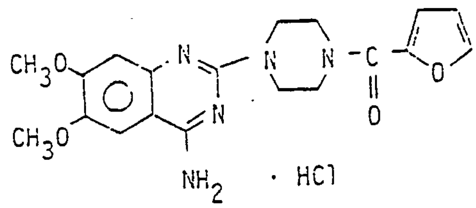

2 g (6.91 mmol) 4-Amino-6,7-dimethoxy-2-piperazinochinazolin werden in 21 ml Ethanol (p. A.) suspendiert und unter kräftigem Rühren bei Raumtemperatur mit 0.90 g (6.91 mmol) 2-Furancarbonsäurechlorid versetzt. Nach 3 h wird das ausgefallene Produkt abgesaugt, aus Methanol (p. A.) umkristallisiert und bei 100 °C getrocknet.

Ausbeute : 2;72 g (94 %) — Schmelzpunkt : 276-277 °C

Rf-Wert : 0.62 (Essigester/MeOH/$HN(C_2H_5)_2$ 70 : 20 : 5)

$C_{19}H_{22}O_4N_5Cl$ (419.9)

Ber. : C 54,34 H 5,28 N 16,68 Cl 8,44

Gef. : C 54,48 H 5,18 N 16,41 Cl 8,37

$^1$H-NMR-Daten : $\sigma$ = 3;86 (s, breit) (2 × $OCH_3$) 6 H,

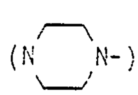

8 H,

($d_6$-DMSO, TMS als interner Standard) 4.03 (m, breit)

6.67 (m) (Aromaten-H) 1 H,
7.13 (d) (Aromaten-H) 1 H,
7.76 (s) (Aromaten-H) 1 H,
7.83 (s) (Aromaten-H) 1 H,
7.93 (s) (Aromaten-H) 1 H,
8.67 (breit) 1 H (austauschbar mit $D_2O$)
9.1 (breit) 2 H (austauschbar mit $D_2O$) ppm.

a) Herstellung von N″-Cyano-N′-(3,4-dimethoxyphenyl)-O-phenylisoharnstoff

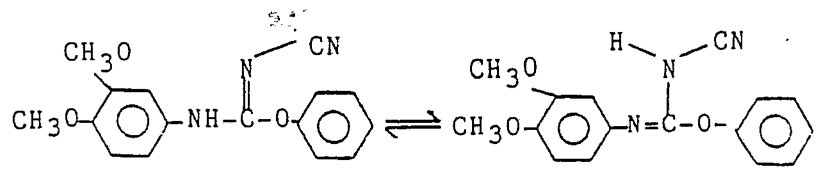

7.66 g (50 mmol) 3,4-Dimethoxyanilin und 11.91 g (50 mmol) N-Cyanodiphenylimidocarbonat werden in 50 ml Isopropanol gelöst und 80 Min. auf Rückflußtemperatur erhitzt. Anschließend läßt man unter Rühren abkühlen und saugt die anfallenden Kristalle ab.

Ausbeute : 13.8 g (93 %) — Schmelzpunkt 163-164 °C (Zers.)
Rf-Wert : 0.83 ($CH_2Cl_2$/MeOH 9 : 1)
$C_{16}H_{15}N_3O_3$ (297)
Ber. : C 64.64 H 5.09 N 14.13
Gef. : C 64,80 H 5.06 N 13.61
$^1$H-NMR-Daten : (d$_6$-DMSO, TMS als interner Standard)
σ = 3.80 (s) (2 × $OCH_3$) 6 H,
6.83-7.67 (m) (Aromaten-H) 8 H,
10,5 (s) (N-H) (austauschbar mit $D_2O$) 1 H ppm.

b) Herstellung von Piperazino-1-[N-cyano-N′-(3,4-dimethoxyphenyl)] carboximidamid

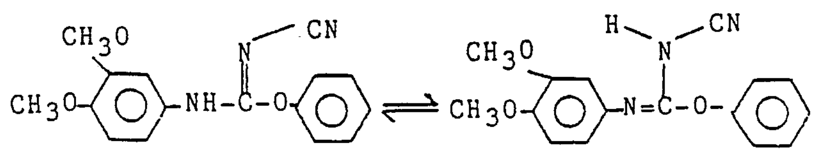

2,50 g (8.4 mmol) N″-Cyano-N′-(dimethoxyphenyl)-O-phenylisoharnstoff und 2.17 g (25.2 mmol) Piperazin werden 30 Minuten in Isopropanol auf Rückflußtemperatur erwärmt. Dabei fällt das Produkt kristallin und DC-rein an.

Ausbeute : 2,18 g (90 %) — Schmelzpunkt : 181-182 °C
Rf-Wert : 0.24 (MeOH techn.)
$C_{14}H_{19}N_5O_2$ (289)
$^1$H-NMR-Daten : (d$_6$-DMSO, TMS als interner Standard)

σ = 2.67 (m, breit) 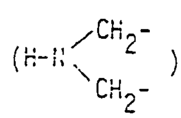 4 H,

3.40 (m, breit) 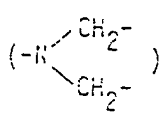 4 H,

3.73 (s) (2 × $OCH_3$) 6 H,
6.50-7.00 (m) (Aromaten-H) 3 H,
9.00 (breit) (NH) 1 H (austauschbar mit $D_2O$) ppm.

c) Herstellung von 4-Amino-6,7-dimethoxy-2-piperazinochinazolin

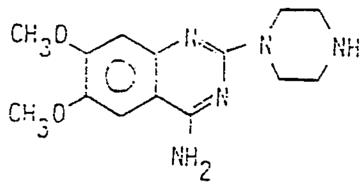

Eine Suspension von 15 g (51.8 mmol) Piperazino-1-[N″-cyano-N′-(3,4-dimethoxyphenyl] carboximidamid wird in 200 ml 10 %iger wäßriger HCl-Lösung 1,5 h auf Rückflußtemperatur erhitzt. Nach dem Abkhühlen wird das Hydrochlorid abfiltriert, mit EtOH gewaschen und getrocknet. (Schmp. 270-275 °C) Das Salz wird in 300 ml 2nNaOH 30 Minuten suspendiert, das Produkt abfiltriert und aus 95 % EtOH umkristallisiert.

Ausbeute : 11,2 g (75 %) — Schmelzpunkt : 230-232 °C
Rf-Wert : 0.07 (MeOH techn.)
$C_{14}H_{19}N_5O_2$ (289)
$^1$H-NMR-Daten :
($d_6$-DMSO, TMS als
interner Standard)

$\sigma$ = 2.67 (m, breit) 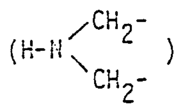 4 H,

3.45 (breit) (= N—H) 1 H (austauschbar mit $D_2O$)

3.60 (m, breit) 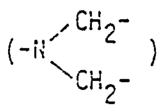 4 H,

3.73 (s) (—$OCH_3$) 3 H,
3.78 (s) (—$OCH_3$) 3 H,
6.63 (s) (Aromaten-H) 1 H,
7.04 (s) breit (—$NH_2$) 2 H, (austauschbar mit $D_2O$)
7.37 (s) (Aromaten-H) 1 H ppm.

Das Eintopfverfahren ergab eine Gesamptausbeute von 4-Amino-6,7-dimethoxy-2-piperazinochinazolin von 78 % der Theorie.

**Patentansprüche**

1. Verfahren zur Herstellung von 4-Amino-6,7-dimethoxy-2-[4-(furo-2-yl)-piperazin-1-yl]-chinazolin der Formel I

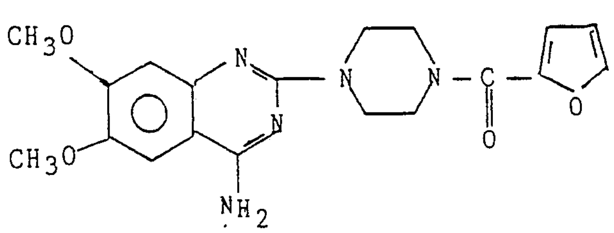

(I)

und dessen physiologisch annehmbarer Salze, dadurch gekennzeichnet, daß man

a) 3,4-Dimethoxyanilin der Formel II

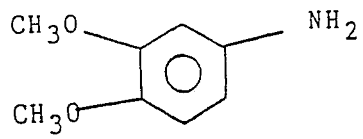

(II)

mit äquimolaren Mengen N-Cyanodiphenylimidocarbonat der Formel III

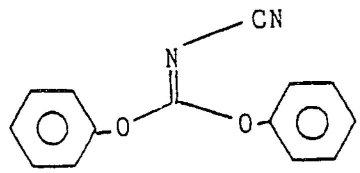

(III)

in einem Alkanol als Lösungsmittel, wobei die Reaktionstemperatur zwischen 20 °C und Rückflußtemperatur des Lösungsmittels beträgt und die Reaktionsdauer, je nach Temperatur, 1 bis 2 Stunden beträgt, zu N''-Cyano-N'-(3,4-dimethoxyphenyl)-O-phenylisoharnstoff der tautomeren Formeln IV

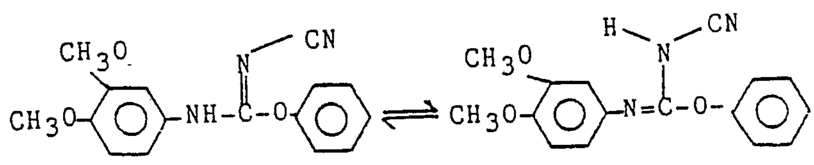

(IV)

umsetzt ;

b) die erhaltene Verbindung der tautomeren Formeln IV mit Piperazin im Überschuß in einem Alkanol, wobei die Reaktionstemperatur zwischen 20 °C und 82 °C und die Reaktionszeit 0,5 bis 1 Stunde beträgt, zu dem Piperazino-1-N''-cyano-N'-(3,4-dimethoxyphenyl) carboximidamid der tautomeren Formeln V

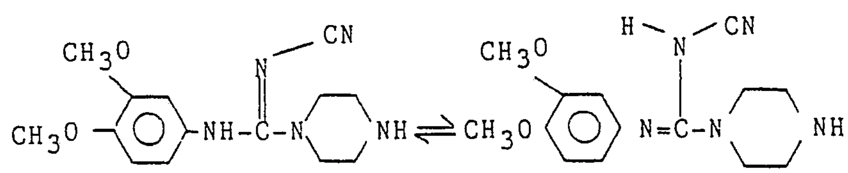

(V)

umsetzt ;

c) anschließend mit einer Säure unter sauren Bedingungen bei einer Temperatur von 20 °C bis 100 °C und einer Reaktionsdauer von 20 bis 120 Minuten zu dem Salz des 4-Amino-6,7-dimethoxy-2-piperazinochinazolins der Formel VI

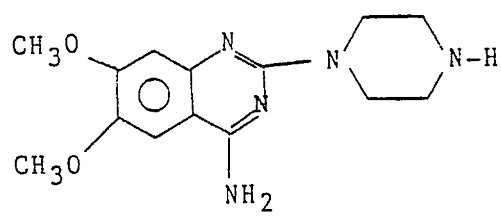

(VI)

cyclisiert ;

d) aus dem in Stufe c) erhaltenen Salz durch Umsetzung mit einem 2- bis 12-fachen molaren Überschuß von wäßrigem Alkali bei Raumtemperatur die freie Base VI gewinnt ;

e) die freie Base mit 2-Furancarbonsäurechlorid in einem Alkanol bei 25 °C zu 4-Amino-6,7-dimethoxy-2 [4-(furo-2-yl)-piperazin-1-yl]-chinazolin-Hydrochlorid umsetzt ; und gegebenenfalls

f) das in Stufe e) erhaltene Hydrochlorid in an sich bekannter Weise in ein anderes physiologisch annehmbares Salz umwandelt.

2. Verfahren nach Anspruch 1, dadurch gekennzeichnet, daß man die Stufen a) bis c) in einem « Eintopfverfahren » durchführt.

3. Verfahren nach Anspruch 2, dadurch gekennzeichnet, daß man in Stufe c) die Cyclisierung in einer 10 %igen wäßrigen Salzsäurelösung durchführt.

4. Verfahren nach Anspruch 2, dadurch gekennzeichnet, daß man in Stufe c) die Cyclisierung in einer 10 %igen isopropanolischen Salzsäurelösung durchführt.

5. Verfahren nach Anspruch 1, dadurch gekennzeichnet, daß man in Stufe d) als Alkali wäßrige Natronlauge verwendet.

6. Verfahren nach Anspruch 1, dadurch gekennzeichnet, daß man in Stufe e) die Acylierungsreaktion in Ethanol durchführt.

7. N"-Cyano-N'-(3,4-dimethoxyphenyl)-O-phenylisoharnstoff der tautomeren Formeln IV

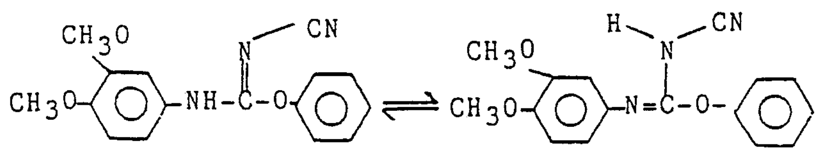

(IV)

**Claims**

1. Process for the preparation of [4-amino-6,7-dimethoxy-2-4-(furo-2-yl)-piperazin-1-yl]-quinazoline corresponding to Formula I

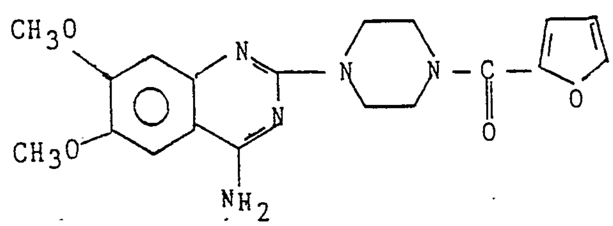

(I)

and its physilogically acceptable salts, characterised in that

a) 3,4-dimethoxyaniline corresponding to Formula II

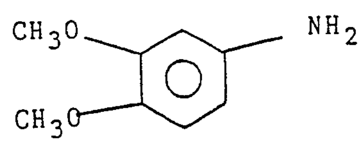

(II)

is reacted with equimolar quantities of N-cyanodiphenylimidocarbonate corresponding to Formula III

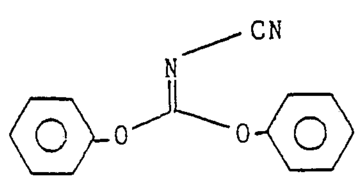

(III)

in an alkanol as solvent at a reaction temperature from 20 °C to the reflux temperature of the solvent and for a reaction time of from 1 to 2 hours, depending on the temperature, to form N"-cyano-N'-(3,4-dimethoxyphenyl)-O-phenyl-isourea corresponding to the tautomeric Formulae IV

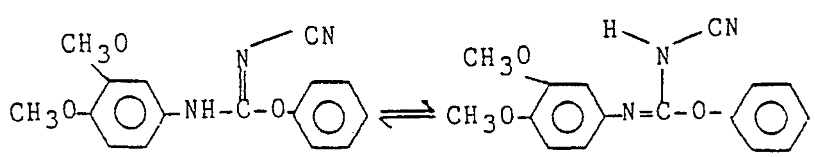

(IV)

b) the resulting compound corresponding to the tautomeric Formulae IV is reacted with an excess

of piperazine in an alkanol at a reaction temperature of from 20 °C to 82 °C and for a reaction time of from 0.5 to 1 hour to form the piperazino-1-N''-cyano-N'-(3,4-dimethoxyphenyl)-carboximidamide corresponding to the tautomeric Formulae V ;

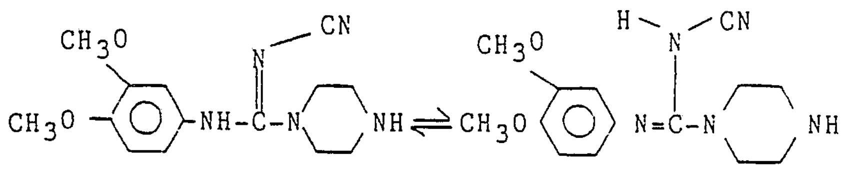

(V)

c) the reaction product obtained is then cyclised with an acid under acid conditions at a temperature of from 20 °C to 100 °C and a reaction time of from 20 to 120 minutes to form the salt of 4-amino-6,7-dimethoxy-2-piperazino-quinazoline corresponding to Formula IV ;

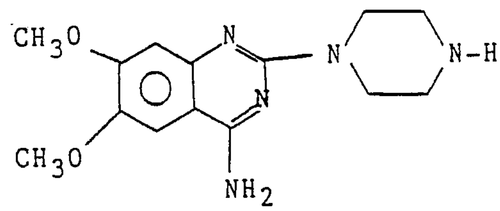

(VI)

d) the free base VI is obtained from the salt obtained in stage c) by reacting said salt with a 2 to 12 × molar excess of aqueous alkali at room temperature ;

e) the free base is reacted with 2-furan-carboxylic acid chloride in an alkanol at 25 °C to form 4-amino-6,7-dimethoxy-2 [4-(furo-2-yl)- piperazin-1-yl]-quinazoline hydrochloride ; and optionally

f) the hydrochloride obtained in stage e) is converted in known manner into another physiologically acceptable salt.

2. Process according to Claim 1, characterised in that stages a) to c) are carried out in a « one shot process ».

3. Process according to Claim 2, characterised in that cyclisation in stage c) is carried out in a 10 % aqueous hydrochloric acid solution.

4. Process according to Claim 2, characterised in that cyclisation in stage c) is carried out in a 10 % isopropanolic hydrochloric acid solution.

5. Process according to Claim 1, characterised in that the alkali used in stage d) is aqueous sodium hydroxide solution.

6. Process according to Claim 1, characterised in that the acylating reaction in stage e) is carried out in ethanol.

7. N''-cyano-N'-(3,4-dimethoxyphenyl)-O-phenyl-isourea corresponding to the tatomeric Formulae IV

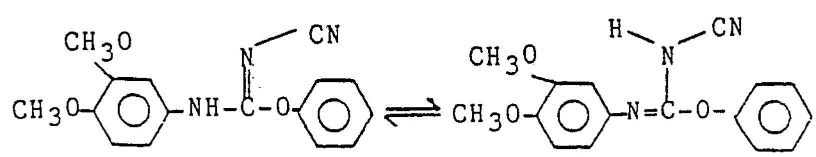

(IV)

**Revendications**

1. Procédé pour la préparation de la 4-amino-6,7-diméthoxy-2-[4-(furo-2-yl)-pipérazin-1-yl]-quinazoline de formule I

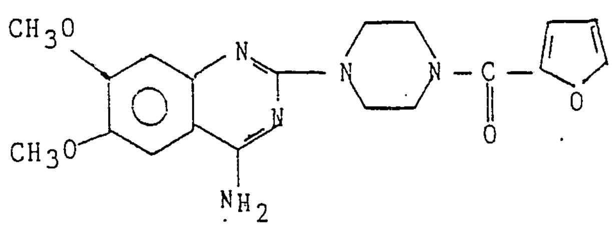

(I)

et ses sels physiologiquement acceptables, caractérisé en ce que :

a) on fait réagir la 3,4-diméthoxyaniline de formule II

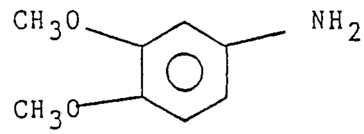

(II)

avec des quantités équimolaires de N-cyano-imidocarbonate diphénylique de formule III

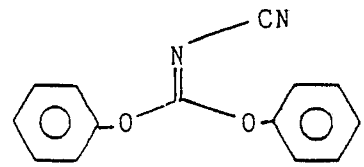

(III)

dans un alcanol comme solvant, la température de réaction s'élevant entre 20 °C et la température de reflux du solvant et la durée de réaction étant, suivant la température, de 1 à 2 heures, de manière à obtenir la N''-cyano-N'-(3,4-diméthoxyphényl)-O-phényl-iso-urée répondant aux formules IV tautomères

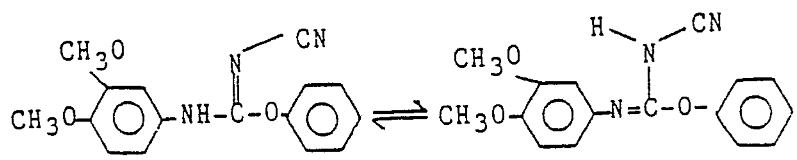

(IV)

b) on fait réagir le composé obtenu répondant aux formules IV tautomères avec la pipérazine en excès dans un alcanol, la température de réaction s'élevant entre 20 °C et 82 °C et le temps de réaction étant de 0,5 à 1 heure, de manière à obtenir le pipérazino-1-N''-cyano-N'-(3,4-diméthoxyphényl) carboximidamide répondant aux formules V tautomères

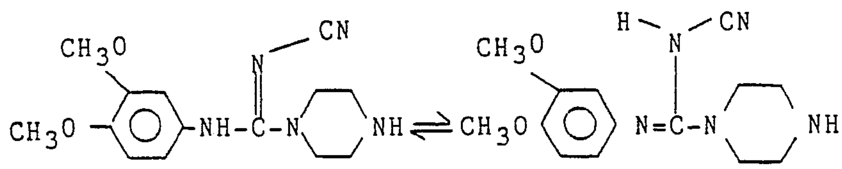

(V)

c) on cyclise consécutivement avec un acide dans des conditions acides à une température de 20 °C à 100 °C et avec une durée de réaction de 20 à 120 minutes, de manière à obtenir le sel de la 4-amino-6,7-diméthoxy-2-pipérazinoquinazoline de formule VI

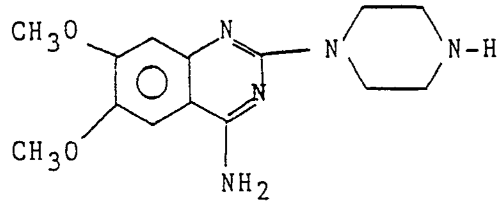

(VI)

d) à partir du sel obtenu au stade c), et par réaction dudit sel avec un excès 2 à 12 fois molaire d'un alcali aqueux à la température ambiante, on isole la base VI libre ;

e) on fait réagir la base libre avec le chlorure d'acide 2-furannecarboxylique dans un alcanol à 25 °C, de manière à obtenir le chlorhydrate de 4-amino-6,7-diméthoxy-2 [4-(furo-2-yl)-pipérazin-1-yl]-quinazoline ; et éventuellement

f) on transforme d'une manière connue en soi le chlorhydrate obtenu dans le stade e) en un autre sel physiologiquement acceptable.

2. Procédé selon la Revendication 1, caractérisé en ce qu'on met en œuvre les stades a) à c) en un « mode opératoire dit en un seul pot ».

3. Procédé selon la Revendication 2, caractérisé en ce que dans le stade c), on effectue la cyclisation dans une solution aqueuse d'acide chlorhydrique à 10 %.

4. Procédé selon la Revendication 2, caractérisé en ce que dans le stade c), on effectue la cyclisation dans une solution isopropanolique d'acide chlorhydrique à 10 %.

5. Procédé selon la Revendication 1, caractérisé en ce que dans le stade d), on utilise comme alcali une lessive aqueuse de soude caustique.

6. Procédé selon la Revendication 1, caractérisé en ce que dans le stade e), on effectue la réaction d'acylation dans de l'éthanol.

7. N''-Cyano-N'-(3,4-diméthoxyphényl)-O-phényl-iso-urée répondant aux formules IV tautomères.

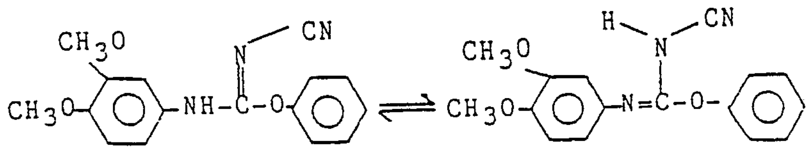

(IV)